# EUROPEAN PATENT APPLICATION

(11) **EP 1 932 906 A1**
(43) Date of publication of application: **18.06.2008**
(21) Application number: 06810758.0
(22) Date of filing: 28.09.2006
(51) Int. Cl.: C12N 5/10, A01K 67/02, C12N 15/09

(54) **METHOD FOR PRODUCING NUCLEAR-TRANSPLANTED EGG**

(30) Priority: 30.09.2005 JP 2005287361; 31.07.2006 JP 2006208565
(71) Applicant: Riken, Wako-shi, Saitama 351-0198 (JP); Kinki University, Higashiosaka-shi Osaka 577-8502 (JP); Japan Science and Technology Agency, Kawaguchi-shi, Saitama 332-0012 (JP)
(72) Inventor: KISHIGAMI, Satoshi, Kobe-shi, Hyogo 650-0047 (JP); WAKAYAMA, Teruhiko, Kobe-shi, Hyogo 650-0047 (JP); SAEKI, Kazuhiro, Kinokawa-shi, Wakayama 649-6493 (JP)
(74) Representative: Schlich, George William
(86) International application number: PCT/JP2006/319311
(87) International publication number: WO 2007/043346

(57) **Abstract**

It is intended to provide a method for improving a development rate in a somatic nuclear transplantation technique or an artificial insemination technique using a spermatid. The method is a method for producing a nuclear-transplanted egg comprising the steps of transplanting a nucleus of a donor cell into an egg, and treating the nuclear-transplanted egg with an anti-methylating agent.

## Description

### Technical Field

The present invention relates to a method for producing a nuclear transfer oocyte. Specifically, the present invention relates to a method by which the development rate in a somatic nuclear transfer or an artificial fertilization using a spermatid is increased.

### Background Art

Recently, successful production of cloned animals using somatic nuclear transfer has been reported for various species (sheep, mouse, cow, etc.). Furthermore, it has become possible to establish mouse-derived NT-ES cells (nuclear transfer embryonic stem cells), i.e., ES cells derived from somatic cells, by culturing cloned embryos obtained by means of somatic nuclear transfer.

The most significant problem associated with conventional somatic nuclear transfer technologies was the low development rate of embryos following nuclear transfer. Based on later studies, extraordinarily high modification by methylation of DNA and histone in cloned embryos as compared with conventional fertilized oocytes has been reported, and has been considered to be the cause of the low development rate.

To date, attempts have been made to increase the development rate of nuclear transfer embryos (cloned embryos) which has been low due to the hypermethylation. For example, it has been reported that a blastocyst development rate was increased by culturing, prior to nuclear transfer, fibroblasts as donor cells in the presence of an agent such as trichostatin A (a histone deacetylase inhibitor) or 5'-aza-2'-deoxycytidine (a DNA methyltransferase inhibitor) (Non-patent Document 1). Furthermore, it has been reported that a blastocyst development rate was increased by subjecting immortalized bovine mammary epithelial cells (MECL) or bovine fetal fibroblasts (BFF) to nuclear transfer following treatment with sodium butyrate (a histone deacetylase inhibitor) (Non-patent Document 2). These studies were made aiming to increase the development rate by placing, prior to the nuclear transfer, the nuclei from donor cells in a state more similar to that of fertilized oocytes and then reprogramming the nuclei. However, the effect was small and the improvement has not reached a practical level.

Recently, assisted reproductive technologies have been developed, and a nuclear transfer technology called ROSI (round spermatid injection) has been developed. It has been reported that humans or mice could be produced using this technology for artificial fertilization with spermatids, which are sperm precursors lacking the fertilizing ability by nature. However, the development rate of embryos obtained using spermatids is low, and the cause has been unknown.

Somatic cell clone technology is very useful for increasing production of particular cows having high economically important traits or for efficient production of transgenic cows useful for production of medicines or the like. However, the development rate of bovine cloned embryos is very low (Non-patent Documents 16 and 17). If the development rate of bovine cloned embryos can be improved, the female embryo recipients miscarriage rate will be reduced, and the production efficiency will be remarkably increased. Methods for improving a development rate include a method in which the cell cycle of donor somatic cells is adjusted at the G1 phase (Non-patent Documents 18 and 19, Patent Document 1), and a method in which embryos with high development efficiency are selected by examining the gene expression in the produced cloned embryos (Patent Document 2). However, these methods require considerable skill for adjusting the cell cycle, or special equipment for examining gene expression. Thus, it has been desired to increase the development rate of bovine cloned embryos using an easy method and an easy technique.

Patent Document 1: JP-A 2003-052369
Patent Document 2: JP-A 2006-166828
Non-patent Document 1: Enright, B.P. et al., Biol. Reprod., 69:896-901 (2003)
Non-patent Document 2: Shi, W. et al., Biol. Reprod., 69:301-309 (2003)
Non-patent Document 3: Ma, J. et al., Biol. Reprod., 64:1713-1721 (2001)
Non-patent Document 4: Chen, Y. et al., Cell Research, 13:251-263 (2003)
Non-patent Document 5: Wakayama, T. et al., Nature, 394:369-374 (1998)
Non-patent Document 6: Kishigami, S. et al., Biol. Reprod., 70:1863-1869 (2004)
Non-patent Document 7: Christman, J.K., Oncogene, 21:5483-5495 (2002)
Non-patent Document 8: De Ruijter, A.J.M. et al., Biochem. J., 370:737-749 (2003)
Non-patent Document 9: Santos, F. et al., Curr. Biol., 13:1116-1121 (2003)
Non-patent Document 10: Wakayama, T. et al., J. Reprod. Fertil., 112:11-17 (1998)
Non-patent Document 11: Wakayama, T. et al., Nat. Genet., 22:127-128 (1999)
Non-patent Document 12: Hooper, M. et al., Nature, 326:292-295 (1987)
Non-patent Document 13: Kishigami, S. et al., Biol. Reprod., 70:1863-1869 (2004)
Non-patent Document 14: Kishigami et al., Zygote, 12:321-327 (2004)
Non-patent Document 15: Dean, W. et al., Proc. Natl. Acad. Sci. USA, 98:13734-13738 (2001)
Non-patent Document 16: Hill, J.R. et al., Biol. Reprod., 63:1787-1794 (2000)
Non-patent Document 17: Heyman, Y., et al., Biol. Reprod., 66:6-13 (2002)
Non-patent Document 18: Kasinathan, P. et al., Nat. Biotechnol., 12:1176-1178 (2001)
Non-patent Document 19: Urakawa, M. et al., Theriogenology, 62:714-728 (2004)
Non-patent Document 20: Robl, J.M. et al., J. Anim. Sci., 64:642-647 (1987)
Non-patent Document 21: Tervit, H.R. et al., J. Reprod. Fertil., 30:493-497 (1972)
Non-patent Document 22: Takahashi, Y. et al., Theriogenology, 37:963-978 (1992)

### Disclosure of Invention

### Problems to be Solved by the Invention

The present inventors have intensively studied for solving the above-mentioned problems. The present inventors have found the following: hypermethylation takes place in oocytes after nuclear transfer in studies using mice as model animals and spermatids; hypermethylation can be repressed by treating nuclear transfer oocytes with an anti-methylation agent after nuclear transfer; the in vitro development rate of cloned embryos and the birth rate of cloned animals are improved by this treatment; and the same treatment is also effective in the improvement of the development of embryos obtained using spermatids. Thus, the present invention has been completed. It is possible to greatly improve the low development rate of nuclear transfer embryos not by treating donor cells with an anti-methylation agent before nuclear transfer as in the prior art, but by conducting treatment with an anti-methylation agent after transferring nuclei into oocytes to repress hypermethylation of DNA.

It was known that the development rate is reduced by treatment of oocytes with a histone deacetylase inhibitor trichostatin A, which is an anti-methylation agent, after fertilization (Non-patent Document 3). Thus, the above-mentioned finding by the present inventors was unexpected.

The main object of the present invention is to provide a method by which a development rate in a somatic nuclear transfer or an artificial fertilization using a spermatid is increased.

### Means to Solve the Problems

The present invention relates to:
[1] A method for producing a nuclear transfer oocyte, the method comprising:
   (a) transferring a nucleus from a donor cell into an oocyte to obtain a nuclear transfer oocyte; and
   (b) treating the nuclear transfer oocyte with an anti-methylation agent;
[2] The method according to [1], wherein the treatment in step (b) is carried out during a period after the transfer of the nucleus and before embryo implantation;
[3] The method according to [1], wherein the treatment in step (b) is carried out during a period after the transfer of the nucleus and before the end of the blastocyst stage;
[4] The method according to [1], wherein the treatment in step (b) is carried out during a period after the transfer of the nucleus and before the end of the 32-cell stage;
[5] The method according to [1], wherein the treatment in step (b) is carried out during a period after the transfer of the nucleus and before the end of the 4-cell stage;
[6] The method according to [1], wherein the treatment in step (b) is carried out during a period after the transfer of the nucleus and before zygotic gene activation;
[7] The method according to [1], wherein the treatment in step (b) is carried out during a period after the transfer of the nucleus and before the end of remethylation;
[8] The method according to [1], wherein the anti-methylation agent is a histone deacetylase inhibitor;
[9] The method according to [8], wherein the histone deacetylase inhibitor is trichostatin A or apicidin;
[10] The method according to [1], wherein the oocyte is an enucleated oocyte;
[11] The method according to [10], wherein the donor cell is selected from the group consisting of a cumulus cell, a fibroblast and an ES cell;
[12] The method according to [1], wherein the donor cell is a spermatid;
[13] A method for producing a cloned animal, the method comprising:
   (a) transferring a nucleus from a donor cell into an oocyte to obtain a nuclear transfer oocyte;
   (b) treating the nuclear transfer oocyte with an anti-methylation agent; and
   (c) implanting the nuclear transfer oocyte treated with the anti-methylation agent into an animal; and
[14] A nuclear transfer oocyte produced according to the method defined by [1].

### Effects of the Invention

The method of the present invention is useful for production of a cloned animal, establishment of an ES cell from a cloned embryo (NT-ES cell), and improvement of nuclear transfer technologies which are used for a wide variety of purposes, such as infertility treatment using a spermatid.

### Brief Description of Drawings

Figure 1 shows blastocyst formation upon treatments with trichostatin A at varying concentrations. Left upper: 0 nM; right upper: 0.5 nM; left lower: 5 nM; right lower: 50 nM.
Figure 2 illustrates blastocyst formation rates upon treatments with trichostatin A at varying concentrations.
Figure 3 shows blastocyst formation upon treatments with trichostatin A for varying periods of time. Left upper: 0 hour; right upper: 24 hours; left lower: 48 hours; right lower: 72 hours.
Figure 4 illustrates blastocyst formation rates upon treatments with trichostatin A for varying periods of time.
Figure 5 illustrates blastocyst formation rates upon treatments with trichostatin A for varying periods of time.
Figure 6 shows cloned mouse fetuses and placentas immediately after birth (left), and black cloned mice with a white surrogate mother after three weeks (right).
Figure 7 shows blastocyst formation in oocytes into which nuclei from various donor cells were transferred. Left: no TSA treatment; right: treatment with 50 nM TSA.
Figure 8 illustrates blastocyst formation rates of oocytes into which nuclei from various donor cells were transferred. Shaded bars: cumulus cells; black bars: fibroblasts; white bars: ES cells.
Figure 9 illustrates the influence of TSA treatments on methylation of DNA and histone.

### Best Mode for Carrying Out the Invention

The method for producing a nuclear transfer oocyte of the present invention comprises:
(a) transferring a nucleus from a donor cell into an oocyte to obtain a nuclear transfer oocyte; and
(b) treating the nuclear transfer oocyte with an anti-methylation agent.

Furthermore, a cloned animal is produced according to the present invention by implanting the nuclear transfer oocyte treated with the anti-methylation agent produced according to the above-mentioned method into an animal. The present invention also provides a nuclear transfer oocyte produced according to the above-mentioned method.

According to the present invention, any oocyte that has an ability to develop can be used as an oocyte. An oocyte can be collected according to a conventional method. For example, an oocyte can be collected from an animal that has been subjected to a superovulation treatment. If a somatic cell or the like is to be used as a donor cell, it is preferable to remove a nucleus from an oocyte (enucleate) beforehand. On the other hand, the enucleation is not necessary if a spermatid is to be used as a donor cell as is the case with ROSI. An oocyte is normally activated upon fertilization with a sperm to initiate development, whereas artificial activation is required in cases where a sperm is not used. For example, an oocyte is activated prior to, at the time of, or following transfer (injection) of a nucleus from a donor cell. An oocyte can be activated by culturing the oocyte in the presence of strontium chloride (SrCl₂). Depending on the cell cycle of the donor nucleus, an agent such as cytochalasin B may be added in order to destroy the cytoskeleton to prevent the polar body emission. In the case of a normal fertilized ovum, activation of an oocyte is confirmed based on emission of the second polar body and formation of two (male and female) pronuclei. On the other hand, activation of an oocyte is judged based only on the formation of pronuclei in the case of a cloned embryo because the polar body emission is repressed using an agent as described above.

According to the present invention, any cell that has a nucleus can be used as a donor cell. Examples of donor cells include somatic cells such as cumulus cells, fibroblasts, nerve cells and blood cells (e.g., T cells), germ cells such as spermatids, as well as ES cells. For nuclear transfer according to the present invention, for example, a nucleus obtained from a somatic cell may be injected into an oocyte, or a nuclear domain of a spermatid may be injected into an oocyte.

The donor cell and the oocyte may be derived from the same biological species, or they may be derived from different biological species. For example, a combination of a donor cell and an oocyte derived from the same mammalian species are preferably used according to the present invention. Alternatively, it is possible to inject a nucleus from a biological species different from the oocyte. In this connection, a rabbit or bovine oocyte is known to have an ability to receive a heterologous somatic nucleus, and the establishment of an NT-ES cell by injecting a nucleus from a human donor cell into a rabbit oocyte has been reported (Non-patent Document 4). Such nuclear transfer between different species enables, for example, substitution of an oocyte from a species other than human for a human oocyte to compensate for a shortage of human oocytes for production of human NT-ES cells.

A nucleus from a donor cell can be injected into an oocyte according to a known method. For example, injection of a somatic nucleus into an enucleated oocyte can be carried out according to the method as described in Non-patent Document 5. Injection of a nuclear domain of a spermatid into an oocyte can be carried out according to the method as described in Non-patent Document 6.
Alternatively, cell fusion between a donor cell and an enucleated oocyte can be used to inject a nucleus from the donor cell into the oocyte as described in Patent Document 2. Furthermore, it is possible to produce a cloned animal by implanting a nuclear transfer embryo into an oviduct of an animal according to a known method as described in the above-mentioned literatures.

Any anti-methylation agent can be used according to the present invention. An anti-methylation agent is an agent that directly or indirectly inhibits methylation of DNA. For example, a methylating enzyme inhibitor or a histone deacetylase inhibitor can be used as an anti-methylation agent. In particular, it is preferable to use a histone deacetylase inhibitor. 5'-azacytidine, 5'-aza-2'-deoxycytidine or the like can be used as a methylating enzyme inhibitor. 5-azacytidine is known to be a potent inhibitor of a methylating enzyme, DNA methyltransferase (Non-patent Document 7). Trichostatin A, apicidin or the like can be used as a histone deacetylase inhibitor. In addition, various agents that can be used as histone deacetylase inhibitors are known (Non-patent Document 8). According to the present invention, such an agent may be used alone or any combination thereof may be used.

The concentration of the anti-methylation agent in a medium can be appropriately determined using the effect of the present invention (i.e., increase in development rate) as an index. The concentration of trichostatin A is for example 0.5-500 nM, preferably 5-50 nM. The appropriate concentration can vary depending on the animal species or the somatic cell type to be used. A skilled person in the art can determine the appropriate concentration based on the description of the present specification.

It is preferable to set the period of the treatment with an anti-methylation agent so that gene repression, which is essential for a normal developmental process, is not inhibited. Specifically, it is preferable to carry out the treatment with an anti-methylation agent so that methylation of histone or DNA is repressed upon "reprogramming" (a process of putting a nucleus from a somatic cell or the like back to a state like that of a nucleus from a fertilized oocyte; also called "initialization") of the genome, and to terminate the treatment before the initiation of zygotic gene activation which follows the reprogramming. The treatment with an anti-methylation agent is carried out, for example, during a period after the transfer of the nucleus and before the end of remethylation. The end point of remethylation can be determined based on the methylation level in a developing embryo by measuring 5-methylcytosine over time according to a fluorescent antibody method using an anti-5-methylcytosine antibody. If it is difficult to determine the accurate end point according to this method due to difficulty in quantification, it is desirable to determine the treatment time period that results in the highest rate of development into (expanded) blastocyst by terminating the treatment at an arbitrary time prior to blastocyst. In relation to the developmental stage, the treatment may be initiated after the nuclear transfer and terminated at an arbitrary time prior to blastocyst. In case of a mouse, the treatment with an anti-methylation agent is carried out, for example, during a period after the transfer of the nucleus and before the end of the 1-cell stage so that methylation of histone or DNA is repressed upon reprogramming of genome at the 1-cell stage. A specific period of time for the treatment can also be appropriately determined. In the case of a mouse, the treatment with an anti-methylation agent is carried out during a period after the transfer of the nucleus and until 20 hours after the initiation of oocyte activation, preferably after the transfer of the nucleus and until 10 hours after the initiation of oocyte activation. If the treatment with an anti-methylation agent is carried out too long, gene repression which is essential for normal development (due to remethylation or histone deacetylation) does not appropriately take place. Then, the development may rather be inhibited. The appropriate period of time for the treatment with an anti-methylation agent can vary depending on the animal species or the like to be used. A skilled person in the art can determine the appropriate treatment time period based on the description of the present specification.

There is no specific limitation concerning the timing of the step (b) of treating the nuclear transfer oocyte with an anti-methylation agent as long as it is conducted before the oocyte is subjected to embryo implantation. The timing may be appropriately determined depending on the animal species of interest or the like. For eliminating the possibility that the embryo development efficiency is not sufficiently increased due to improper length of the treatment time period, it is desirable to carry out the treatment during a period preferably before the end of the blastocyst stage, more preferably before the end of the 32-cell stage, still more preferably before the end of the 16-, 8-, 4-, 2- or 1-cell stage.

It appears that the efficiency of treatment with an anti-methylation agent tends to be remarkably increased by carrying out the treatment before zygotic gene activation. Although the reason is not entirely clear, it is known, for example, that trichostatin A interferes with the transcriptionally repressive state of gene expression following the zygotic gene activation to inhibit the subsequent embryo development (Non-patent Document 3). It is assumed that a similar mechanism works in a nuclear transfer embryo. From this point of view, it can be said that it is very preferable to carry out the treatment during a period before the end of the 1-cell stage in case of a mouse, and before the end of the 4-cell stage in case of a cow.

The increase in development rate according to the method of the present invention can be confirmed based on the birth rate of cloned animals (the ratio of the number of born cloned animal fetuses to the number of implanted embryos). Alternatively, the effect of the method of the present invention can be examined using the rate of expanded blastocyst formation upon cultivation of nuclear transfer oocytes (the ratio of the developed blastocysts to the number of nuclear transfer oocytes) as an index. These methods are described in detail in Examples below. For example, development into an expanded blastocyst is confirmed by culturing for 96 hours after the initiation of activation and then observing under a microscope one in which the volume of embryo is increased to result in expansion of the blastocyst and thinning of zona pellucida among blastocysts having blastocoels. It is possible according to the method of the present invention to achieve a blastocyst formation rate of for example at least 30%, preferably 40% or more, more preferably 50% or more, still more preferably 60% or more. Furthermore, it is possible according to the present invention to achieve a birth rate of for example at least 3%, preferably 4% or more, more preferably 10% or more, still more preferably 20% or more. The blastocyst formation rate or the birth rate is increased for example at least by 1.5-fold, preferably by 2-fold or more, more preferably 3-fold or more, still more preferably 5-fold or more as compared with the case without the treatment with an anti-methylation agent.

The degree of methylation of the paternal (i.e., donor cell-derived) genome in a spermatid-nuclear transfer oocyte produced according to the method of the present invention becomes low. The degree of methylation of the paternal genome can be expressed as a percentage (%) of the methylation level of the paternal genome to the methylation level of the maternal (i.e., oocyte-derived) genome. The methylation level can be determined by measurement using the above-mentioned anti-5-methylcytidine antibody. For example, the value for a nuclear transfer oocyte produced according to the method of the present invention by injecting a nuclear domain of a spermatid into an oocyte is for example 30-60%, preferably 40-55%. The percentage (%) of the methylation level of the paternal genome to the methylation level of the maternal genome is reduced for example by at least 20%, preferably by 30% or more, more preferably by 40% or more as compared with the case without the treatment with an anti-methylation agent.

If the treatment with an anti-methylation agent is not carried out, high methylation of whole genomic DNA is observed in a spermatid-nuclear transfer oocyte. On the other hand, low methylation of DNA in chromosomal portions other than the pericentromeric region is observed in a spermatid-nuclear transfer oocyte produced according to the method of the present invention which comprises the treatment with an anti-methylation agent. As to a somatic cell clone, if the treatment with an anti-methylation agent is not carried out, high methylation of whole genomic DNA and high methylation of histone H3 Lys9 are observed, and both of them are reduced by the treatment with an anti-methylation agent. Thus, it is possible to characterize a nuclear transfer oocyte produced according to the method of the present invention based on the low methylation of genomic DNA (in particular, in the portions other than the pericentromeric region) or the low methylation of histone H3 Lys9.

The following Examples illustrate the present invention in more detail, but are not to be construed to limit the scope thereof.

### Example 1

Production of cloned mice including production of nuclear transfer embryos was carried out according to the method as described in Non-patent Document 5. Briefly, pregnant mare serum gonadotrophin (PMSG) was first administered to 8-weeks-old female B6D2F1 (C57BL/6 x DBA/2) mice (Japan SLC) for superovulation treatment. Human choriogonadotropin (hCG) was administered after 48 hours. Oocytes were collected after 16 hours. Nuclei were removed from the collected unfertilized oocytes in the presence of 5 µg/ml of cytochalasin B using a micromanipulator (enucleation).

Next, cell membranes of cumulus cells from a B6D2F1 mouse as donor somatic cells suspended in a 1.2% polyvinylpyrrolidone (PVP) solution were destroyed using an injection pipette, and the nuclei were injected into the enucleated oocytes using a micromanipulator (nuclear transfer). A piezo drive apparatus (Prime Tech) was used to generate piezo-pulses for penetration of zona pellucida or cell membranes which is required for injection of nuclei.

The thus obtained nuclear transfer oocytes were cultured in a CO₂ incubator at 37°C for 30 minutes to 1 hour. The oocytes were then transferred to an oocyte activation solution (Ca²⁺-free CZB medium supplemented with 5 mM strontium chloride (SrCl₂) and 5 µg/ml of cytochalasin B (Non-patent Document 10)) containing 0.5, 5, 50 or 500 nM trichostatin A (hereinafter referred to as TSA) to artificially activate the oocytes and initiate the development. Activation of oocytes was confirmed 6 hours after the initiation of activation using pronuclei formation as an index. Activated nuclear transfer oocytes were transferred to KSOM medium (Specialty Media) containing TSA at the same concentration, and cultured for further 14 hours (for 20 hours after the initiation of activation) in the medium. For a control, dimethyl sulfoxide (DMSO), which was used for dissolving TSA, was used in place of TSA to produce the same concentration.

Then, the nuclear transfer embryos at the 2-cell stage were cultured in KSOM medium without TSA until 96 hours after the initiation of activation, and development into expanded blastocysts was observed. The development into expanded blastocyst was confirmed by culturing for 96 hours after the initiation of activation and then observing under a microscope one in which the volume of embryo was increased to result in expansion of the blastocyst and thinning of zona pellucida among blastocysts having blastocoels. 80 or more embryos were examined in each of the experimental groups of the respective concentrations. The ratio of the developed blastocysts to the number of nuclear transfer oocytes used (blastocyst formation rate (%)) was determined. The ratio of the developed expanded blastocysts to the number of oocytes that initiated cleavage (i.e., developed into the 2-cell stage) among the reconstructed nuclear transfer oocytes (blastocyst formation rate (%)) was determined.

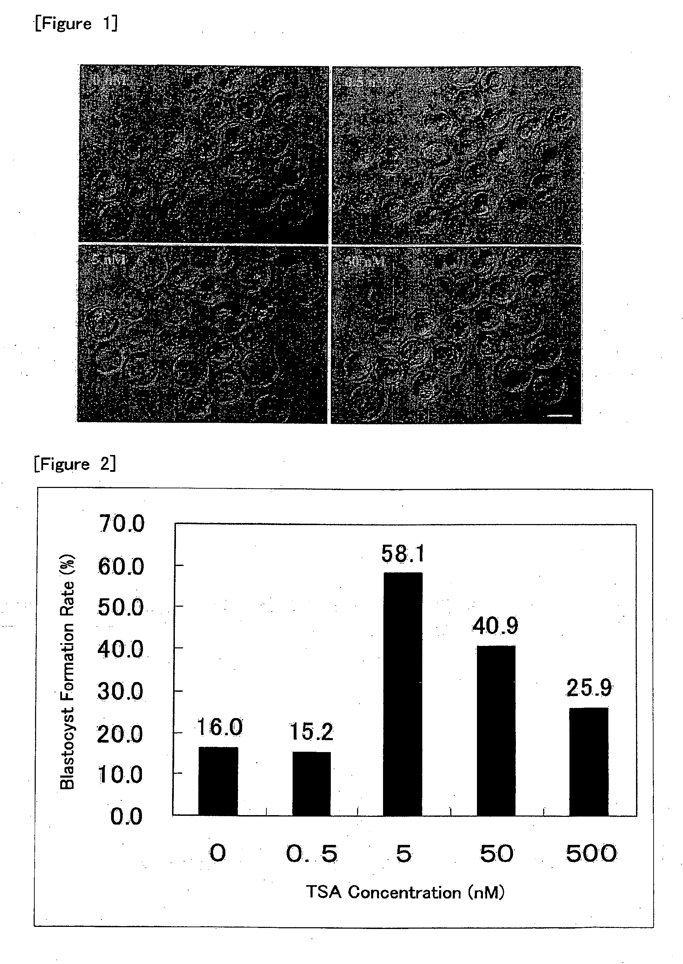

The results are shown in Figures 1 and 2. As shown in Figure 2, the blastocyst formation rate was increased as compared with the case without the treatment with TSA when the treatment with TSA at a concentration of 5, 50 or 500 nM was carried out.

### Example 2

Nuclear transfer oocytes were produced by injecting nuclei from cumulus cells into enucleated oocytes according to the method as described in Example 1, cultured in KSOM medium containing TSA at a concentration of 5 nM for various periods of time (for 0, 24, 48 or 72 hour(s) from the initiation of activation), and then cultured in KSOM medium without TSA until 96 hours after the initiation of activation. Then, development into expanded blastocysts was observed and the blastocyst formation rate was determined.

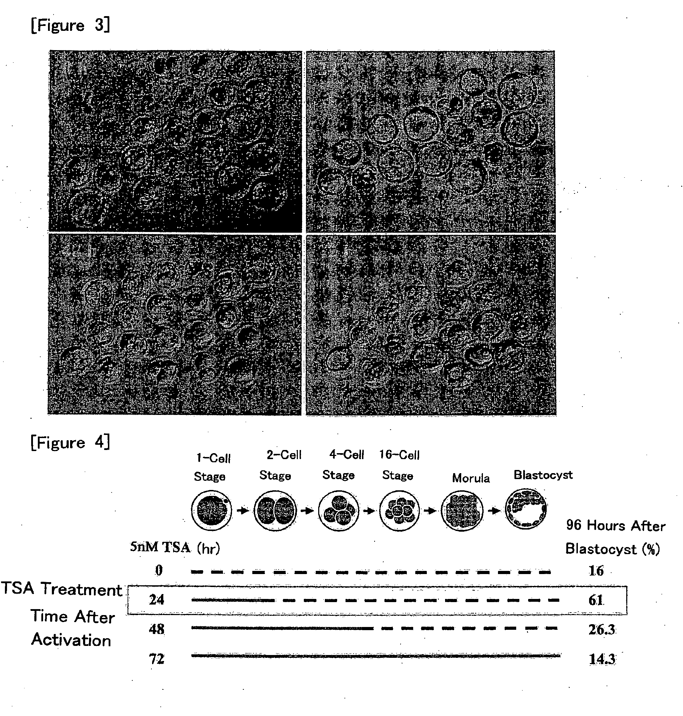

The results are shown in Figures 3 and 4. As shown in Figure 4, the blastocyst development rate was increased as compared with the case without the treatment with TSA when the treatment with TSA was carried out for 24 or 48 hours from the initiation of activation. In particular, about 4-fold increase was observed for the treatment for 24 hours. On the other hand, the development rate was rather reduced when the treatment was carried out for 72 hours from the initiation of activation. 24 hours, 48 hours and 72 hours after the initiation of activation correspond to the 2-cell stage, the 4- to 16-cell stage and the blastocyst formation period, respectively (see Figure 4).

### Example 3

Nuclear transfer oocytes were produced by injecting nuclei from cumulus cells into enucleated oocytes according to the method as described in Example 1, cultured in KSOM medium containing TSA at a concentration of 5 nM for various periods of time (for 0 hour, for 6 hours from the initiation of activation, for 10 hours from the initiation of activation, from 10 to 24 hours after the initiation of activation, or for 24 hours after the initiation of activation), and then cultured in KSOM medium without TSA until 96 hours after the initiation of activation. Then, development into expanded blastocysts was observed and the blastocyst formation rate was determined.

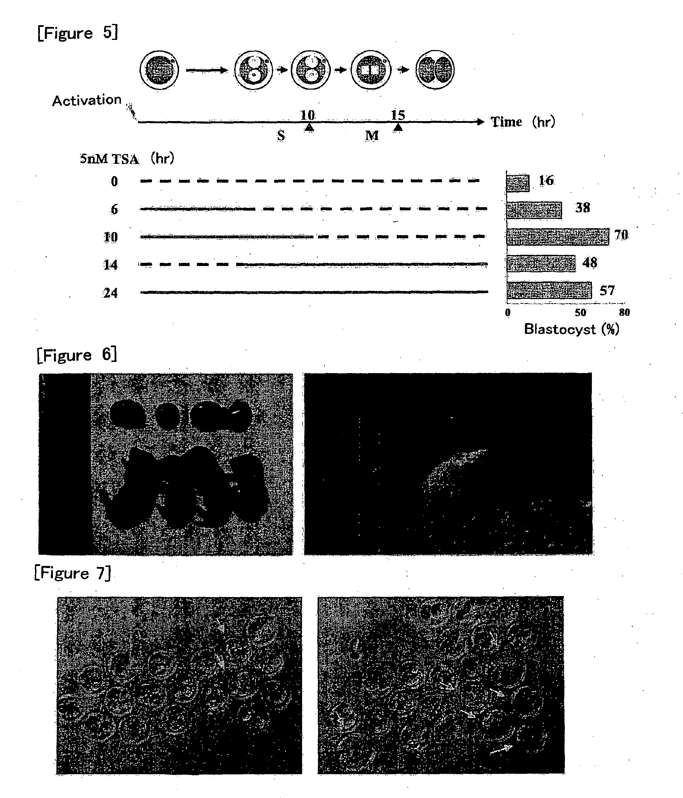

The results are shown in Figure 5. As shown in Figure 5, the highest blastocyst development rate as compared with the case without the treatment with TSA was observed when the treatment with TSA was carried out for 10 hours after the initiation of activation.

### Example 4

Based on the results of Example 3, the effect of the TSA treatment on the fetus birth rate was examined. Nuclear transfer oocytes were treated with TSA at a concentration of 5 nM or 50 nM for 10 hours or 20 hours after the initiation of activation and transferred to KSOM medium without TSA, and the nuclear transfer embryos transferred to KSOM medium without TSA were cultured in this medium until implantation. For producing cloned mice, embryos at the 2-cell stage were implanted into oviducts of pseudopregnant mice (surrogate mothers, day 0.5) on the day after the nuclear transfer. The pseudopregnancy treatment was carried out by mating a ICR female mouse (in estrus) with an ICR vasectomized male mouse on a one-to-one basis. The successful mating was confirmed based on the presence of plug formation. Cloned mice were delivered by cesarean section 19 days after the embryo transfer into oviduct. The ratio of the number of born cloned mouse fetuses to the number of implanted embryos (birth rate) was determined. Cloned mouse fetuses and placentas immediately after birth (left), and cloned mice (black) and a surrogate mother (white) after three weeks (right) are shown in Figure 6. The results are shown in Table 1.

**Table 1**

| TSA conc. (nM) | Treatment time (h) | Number of implanted embryos | Number of born fetuses | Birth rate (%) |
|---|---|---|---|---|
| 0 | 10 | 176 | 2 | 1.1 |
| 5 | 10 | 289 | 18 | 6.2 |
| 5 | 20 | 274 | 9 | 3.2 |
| 50 | 10 | 91 | 7 | 7.7 |
| 50 | 20 | 81 | 2 | 2.5 |

As shown in Table 1, the birth rate was increased by the TSA treatment for 10 hours or 20 hours. In particular, a high birth rate (6-fold higher or more as compared with the control) was observed for the treatment with TSA at a concentration of either 5 nM or 50 nM for 10 hours. 10 hours after the initiation of activation corresponds to the 1-cell stage.

### Example 5

A procedure similar to that described in Example 1 was carried out using fibroblasts or embryonic stem cells (ES cells) as donor cells in place of cumulus cells. Fibroblasts were obtained by culturing a fragment of mouse tail (Non-patent Document 11). E14 cells were used as ES cells (Non-patent Document 12).
Oocytes into which nuclei from the donor cells were transferred were treated with TSA for 20 hours, and cultured in KSOM medium without TSA until 96 hours after the initiation of activation. Then, development into expanded blastocysts was observed and the blastocyst formation rate was determined. 30 or more embryos were examined in each of the experimental groups of the respective cells.

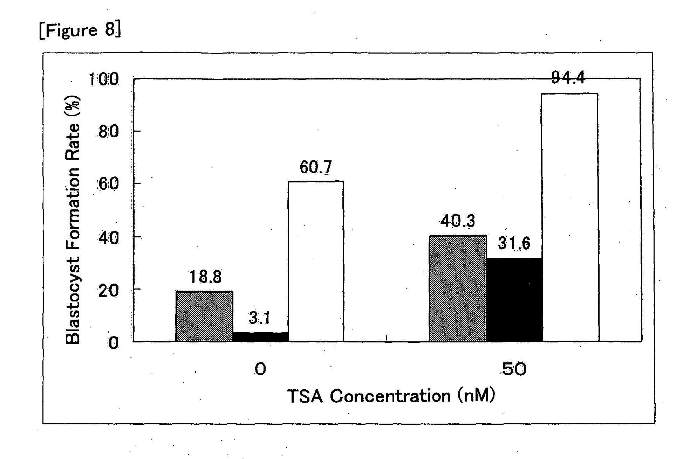

The results are shown in Figures 7 and 8. As shown in Figure 8, higher blastocyst development rates as compared with the case without the treatment with TSA were observed for the nuclei from both of the donor cells being transferred.

### Example 6

Development of nuclear transfer oocytes into expanded blastocysts was observed according to the method as described in Example 1 using 5 µM apicidin (Sigma) in place of TSA. As a result, the blastocyst formation rate for the control was 29.7%, whereas the rate with the apicidin treatment was 69.0%. Thus, the apicidin treatment increased the blastocyst formation rate by about 2-fold.

### Example 7

The effect of the TSA treatment according to the present invention on round spermatid injection (ROSI) was examined. ROSI was carried out according to the method as described in Non-patent Document 13.
Briefly, human choriogonadotropin (hCG) was first administered to female B6D2F1 mice (Japan SLC). Oocytes were collected after 16 hours and placed in a CZB medium. The oocytes were then transferred to a KSOM medium and incubated at 37°C under 5% CO₂.

The oocytes were activated by incubation in Ca²⁺-free CZB medium supplemented with 5 mM strontium chloride (SrCl₂) for 20 minutes. After 40 to 80 minutes, the oocytes were injected with the nuclear domains of round spermatids (characterized by diameters of about 10 µm and centrally located distinct nucleoli; see Non-patent Document 14) collected from a male C57BL/6 mouse (Japan SLC) .

The oocytes injected with nuclei were transferred to KSOM medium containing 5 nM TSA, and further transferred to KSOM medium without TSA 10 hours after the initiation of activation.

Embryos at the 2-cell stage were implanted into oviducts of pseudopregnant mice (surrogate mothers, day 0.5) on the day after the spermatid injection. Fetuses were obtained by cesarean section 19 days after the embryo implantation. The ratio of the number of born fetuses to the number of implanted embryos (birth rate) was determined. The results are shown in Table 2.

**Table 2**

| TSA conc. (nM) | Number of implanted embryos | Birth number | Birth rate (%) |
|---|---|---|---|
| 0 | 34 | 2 | 5.9 |
| 5 | 38 | 10 | 26.3 |

As a result, the birth rate observed for the case with the TSA treatment was increased by about 4-fold as compared with the control without the TSA treatment.

### Example 8

The oocytes injected with the nuclear domains of round spermatids obtained by ROSI were cultured for 20 hours in the presence of 20 ng/ml of demecolcine in a medium containing 10 µM 5-azacytidine (5-aza) or 500 nM TSA as an anti-methylation agent. Similarly, injection of mature sperms was carried out (ICSI (intracytoplasmic sperm injection)). Specifically, heads of sperms were separated from the tails by applying pulses to the head-tail junctions by means of a piezo-driven pipette (PrimeTech), and injected into oocytes. The oocytes were not subjected to activation in this case.

The cultured oocytes injected with nuclei were fixed in PBS containing 4% paraformaldehyde at 4°C overnight, washed in PBS containing 0.1% polyvinyl alcohol (PVA), and blocked in PBS containing 3% bovine serum albumin (BSA) and 0.2% Triton X-100 at 4°C overnight. Subsequent steps were carried out at room temperature. The fixed oocytes injected with nuclei were permeabilized by treating with 0.2% Triton X-100 in PBS at room temperature. The oocytes were then treated with 2N HCl at room temperature for 50 minutes, neutralized in 100 mM Tris-HCl buffer (pH 8.0) (Non-patent Document 15), and extensively washed with PBS containing 1% BSA.

Next, incubation with an anti-5-methylcytidine antibody (Eurogentec) in PBS containing 0.1% PVA was carried out for 2 to 3 hours. After extensively being washed, oocytes were stained with Alexa Flour-488 secondary antibody (Molecular Probes), stained with 5 µg/ml 4',6'-diamidino-2-phenylindole (DAPI) for 30 minutes, and mounted using Vectashield (Vector Laboratory). The specimens were observed using Olympus BX51 microscope (Olympus). All images were captured with DP70 Olympus digital camera using Olympus Analysis software to apply colors to DAPI-stained DNA and merged images.

For quantitative analysis of pronuclear DNA methylation levels, fluorescence images were subjected to densitometric analysis using the program Image-J from the National Institute of Health (http://rsb.info.nih.gov/ij/). For each pronucleus, the relative intensity of methylation of the paternal genome was calculated as a percentage of the fluorescence intensity of the maternal genome. The results are shown in Table 3. In Table 3, data with (b) and without (a) anti-methylation agent were statistically analyzed by Scheffe tests for multiple mean comparisons using the statistical program SPSS version 12.0 (SPSS Inc.). All percentile data were subjected to arcsine transformation prior to the statistical analysis.

**Table 3**

| Injected male germ cell | Antimethylation agent | Number of nuclear transfer oocytes | Ratio of methylation level of paternal genome to maternal genome (%) | ± SEM |
|---|---|---|---|---|
| Mature sperm (ICSI) | No | 52 | 34.9^{a} | 1.5 |
| Mature sperm (ICSI) | 5-aza | 23 | 17.5^{b} | 1.7 |
| Mature sperm (ICSI) | TSA | 13 | 8.2^{b} | 1.1 |
| Spermatid (ROSI) | No | 32 | 77.0^{a} | 2.0 |
| Spermatid (ROSI) | 5-aza | 18 | 54.9^{b} | 3.0 |
| Spermatid (ROSI) | TSA | 23 | 42.5^{b} | 3.3 |

As shown in Table 3, it was confirmed that the methylation levels of the paternal genomes were high when spermatids were injected (ROSI, no anti-methylation agent) as compared with the case where mature sperms were injected (ICSI, no anti-methylation agent). The hypermethylation which is observed upon introduction of spermatids was examined over time. As a result, demethylation took place in both spermatids and mature sperms by 6 hours after the injection, exhibiting lower methylation levels than the maternal genomes that remained highly methylated. However, higher methylation was observed for the cases of injection with spermatids than for the mature sperms 10 hours after the injection. Thus, it was shown that when spermatids were injected, the paternal genomes were first demethylated and then hypermethylated.

As shown in Table 3, the hypermethylation of the paternal genomes, which was observed when spermatids were injected as described above, was reduced using either of the anti-methylation agents.

The DNA methylation observed for the paternal genome upon the treatment with TSA was limited mainly to the pericentromeric region of the chromosome which was represented by high methylation of histone H3 Lys9. Thus, the methylation of the maternal genome was resistant to the TSA treatment. On the other hand, the majority of the methylation of the paternal genome was sensitive to the TSA treatment, while the methylation of DNA in the pericentromeric region which was represented by high methylation of histone H3 Lys9 was resistant to TSA.

### Example 9

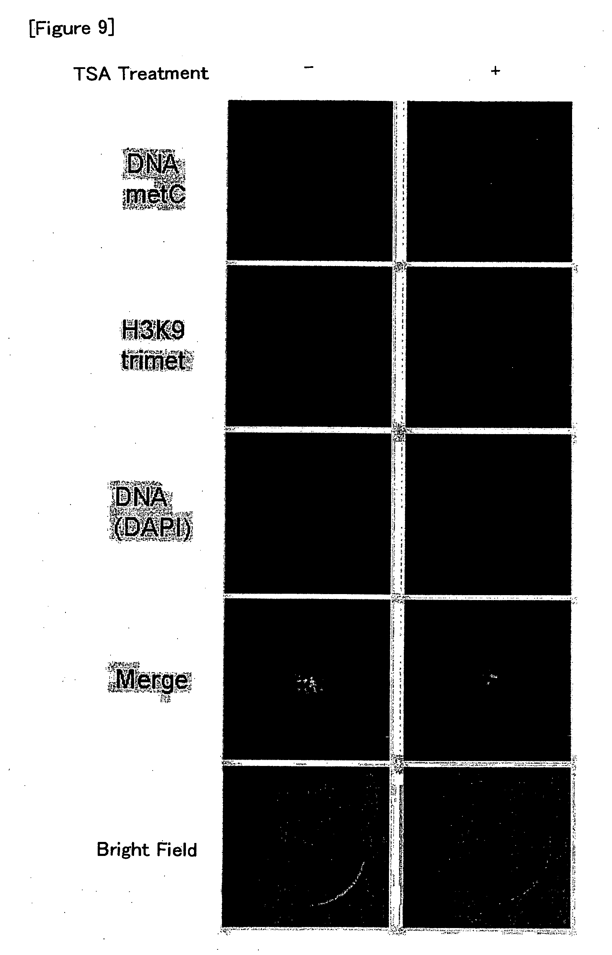

Cloned embryos derived from cumulus cells were produced using oocytes from B6D2F1 mice produced according to the method as described in Example 1, and cultured in the presence or absence of 500 nM TSA for 15 hours (including the activation period). Then, the nuclear transfer embryos were fixed, and methylation of DNA (DNA metC) and methylation of histone H3 Lys9 (H3K9 trimet) were examined using antibodies against the respective antigens. Furthermore, DNA was stained with DAPI. 15 cloned embryos were used for each group. The results are shown in Figure 9 along with the merged images (Merge).

High methylation of both DNA and histone H3 Lys9 was observed over the entire chromosome of the experimental group without the TSA treatment (Figure 9, left, "absence") consistent with the previous report (Non-patent Document 9). As to the experimental group with the TSA treatment (Figure 9, right, "presence"), although prominent reduction was not observed for methylation of either of them in the pericentromeric region, reduction in methylation of both was observed in other chromosomal portions as observed using spermatids in Example 8.

### Example 10

Production of bovine reconstructed embryos and treatment with trichostatin A
(1) Collection of bovine oocytes and in vitro maturation
Bovine ovaries collected from sacrificed cows at a local slaughterhouse were stored in physiological saline at 25°C and transported to the laboratory within 6 to 8 hours after slaughter. Bovine oocytes were harvested by aspiration of follicular fluids from follicles with diameters of 2 to 5 mm on the surfaces of bovine ovaries using 21G injection needles (Terumo, Tokyo, Japan) and 10-ml syringes (Terumo). Only oocyte-cumulus cell complexes to which 2 to 4 layers of cumulus cells were attached were selected and collected from the harvested follicular fluids. The collected oocyte-cumulus cell complexes were introduced into drops of 50 µl maturation medium under mineral oil (Sigma, USA) and cultured for 21 hours at 39°C under 5% CO₂ and 95% air at saturation humidity. The maturation medium was prepared by adding 0.02 AU/ml of follicle-stimulating hormone (FSH, Antrin, Kawasaki Seiyaku, Kanagawa, Japan) and 1 µg/ml estradiol 17-β (Sigma) to 25 mM HEPES buffered TCM-199 (Earle's salts, Gibco, m-TCM199E) supplemented with 5% (v/v) new born calf serum (NBCS, Gibco, NY, USA) and 25 µg/ml of gentamicin solution (Sigma).

(2) Nuclear transfer
(a) Denudation and enucleation of bovine oocytes
The oocyte-cumulus cell complexes matured for 21 hours were allowed to stand for 5 minutes in a solution containing 5% NBCS and 25 µg/ml gentamicin solution (Sigma) in 25 mM HEPES-buffered TCM-199 (Hank's salts, Gibco, m-TCM199H) containing 0.25% (w/v) hyaluronidase (Sigma). Then, the cumulus cells attached around the oocytes were fully removed by pipetting.

The oocytes from which the cumulus cells had been removed were transferred to m-TCM119H and only oocytes with the first polar bodies had been emitted were selected under an inverted microscope. Zonae pellucidae above each first polar bodies were incised using a fine glass needle for removing nuclei from the oocytes, and the oocytes were transferred to m-TCM119H supplemented with 5 µg/ml cytochalacin B (Sigma) and allowed to stand for 15 minutes. Each oocyte was held from the upside using the glass needle used for incising zona pellucida, and enucleation was carried out by removing the first polar body and the oocyte cytoplasm neighboring the first polar body corresponding to about 10 to 20% of the oocyte cytoplasm volume from the zona pellucida incision site. The removed oocyte cytoplasm was transferred to m-TCM199E containing 20 µg/ml of Hoechst 33342 (Sigma) and stained at 39°C under 5% CO₂ and 95% air at saturated humidity for 30 minutes, and the enucleation was confirmed by judging the presence of nucleus in the enucleated oocyte cytoplasm under UV irradiation. Only enucleated oocytes for which nuclei were observed in the removed oocyte cytoplasm were used as recipient enucleated oocytes for the subsequent experiments.

(b) Preparation of donor cells and introduction into enucleated oocytes
Fibroblasts derived from an auricle of Japanese black cattle calves were used as donor cells for nuclear transfer. The donor cells were cultured to 80% confluency in Dulbecco's modified Eagle medium (DMEM, Nissui, Tokyo, Japan) supplemented with 10% (v/v) fetal bovine serum (FBS, Funakoshi, Tokyo, Japan). The culture medium was then exchanged for DMEM supplemented with 0.4% FBS, and the cells were cultured under serum-starvation conditions for 7 days. The cells cultured under serum-starvation conditions were isolated from the culture dishes using a 0.25% (w/v) solution of trypsin (Sigma) containing 0.04%(w/v) disodium dihydrogen ethylenediaminetetraacetic acid (EDTA, Nacalai Tesque, Kyoto, Japan). The cells were harvested by centrifugation in a centrifuge tube, and suspended in a phosphate buffered saline (PBS, Gibco) (-) solution containing 10%(w/v) polyvinylpyrrolidone (PVP, Nacalai Tesque). The isolated cells were aspirated into a micro-glass pipette for cell introduction, and introduced from the zona pellucida incision sites into the perivitelline spaces of the recipient enucleated oocytes.

(c) Electrofusion
A microelectrode (Unique Medical Imada, Miyagi, Japan) was set on a micromanipulator and connected to a BTX cell fusion instrument (ECM200, BTX, Holliston, MA, USA). The recipient enucleated oocytes into which the cells had been introduced were carefully transferred to the Zimmermann cell fusion medium (the composition of the Zimmermann cell fusion medium is shown in Table 4 of Non-patent Document 20). Each enucleated oocyte was held from both polar sides so that the microelectrode, the oocyte cytoplasm and the donor cell were placed in a straight line, and a direct current of 2.7 kV/cm, 11 µsec was applied twice to fuse the enucleated oocyte and the donor cell for producing reconstructed embryos. After the fusion procedure, the reconstructed embryos were transferred to 50-µl drops of m-TCM199E and cultured at 39°C under 5% CO₂, 5% O₂ and 90% N₂ with saturated humidity for 30 minutes. After the cultivation, the fusion between the donor cell and the oocyte cytoplasm was confirmed under a stereoscopic microscope. The cell fusion was confirmed by judging the presence of the donor cells in the perivitelline spaces, and the oocytes for which no cell could be observed were used as reconstructed embryos.

**Table 4: Composition of Zimmermann cell fusion medium**

| Component | Concentration |
|---|---|
| Sucrose^{a} | 280.0 mM |
| Magnesium acetate^{a} | 0.5 mM |
| Calcium acetate^{a} | 0.1 mM |
| Dipotassium hydrogen phosphate^{a} | 1.0 mM |
| Glutathione^{b} | 0.1 mM |
| Bovine serum albumin^{b} | 0.01 mg/ml |

| | |
|---|---|
| a: Nacalai Tesque, b: Sigma | |

(d) Activation
The reconstructed embryos were transferred to a 5 µM solution of ionomycin (Sigma) in Dulbecco's phosphate buffered saline (D-PBS, Gibco) supplemented with 0.1%(w/v) polyvinyl alcohol (PVA, Sigma) and allowed to stand for 5 minutes. The embryos were immediately transferred to 50-µl drops of a culture medium and treated at 39°C under 5% CO₂, 5% O₂ and 90% N₂ at saturation humidity for 6 hours for activation. The culture medium was prepared as follows: the synthetic oviduct fluid medium (SOFM, Non-patent Document 21) was modified by adding 20 essential or non-essential amino acids (Non-patent Document 22); inorganic phosphate was removed therefrom (modified SOFM (mSOFM), the composition of modified synthetic oviduct fluid medium (mSOFM) is shown in Table 5); and 10 µg/ml of cycloheximide (Sigma) was added thereto.

**Table 5: Composition of modified synthetic oviduct fluid medium (mSOFM)**

| Component | Concentration |
|---|---|
| Sodium chloride^{a} | 107.70 mM |
| Potassium chloride^{a} | 7.16 mM |
| Calcium chloride^{b} | 1.71 mM |
| Magnesium chloride^{b} | 1.49 mM |
| Sodium hydrogencarbonate^{a} | 25.07 mM |
| Glucose^{b} | 1.50 mM |
| Sodium pyruvate^{a} | 0.33 mM |
| Sodium lactate^{c} | 3.30 mM |
| L-Glutamine^{c} | 1.00 mM |
| Basal medium eagle 100 x (modified) | 1 % (v/v) |
| amino acids without L-glutamine^{d} | |
| Minimum essential medium 100 x | 1 %(v/v) |
| non-essential amino acids^{d} | |
| Antibacterial antifungal agent solution^{e} | 1 %(v/v) |
| Phenol Red^{e} | 1.0 mg/ml |
| Bovine serum albumin^{c} | 3.0 mg/ml |

| | |
|---|---|
| a: Nacalai Tesque, b: Kanto Chemical, Tokyo, Japan, c: Sigma, d: Dainippon Sumitomo Pharma, Osaka, Japan, e: Gibco | |

(e) In vitro culture
The activated reconstructed embryos were transferred to mSOFM and washed twice. Then, the embryos were transferred to 50-µl drops of the same culture medium at a density of 20 to 30 embryos per drop, and cultured at 39°C under 5% CO₂, 5% O₂ and 90% N₂ at saturation humidity for 168 hours (7 days).

(f) Trichostatin A treatment
The reconstructed embryos were treated with trichostatin A (TSA, Sigma) for 48 hours from the initiation of activation. TSA was dissolved in dimethyl sulfoxide (DMSO, Sigma) and added to the solution for activation medium and mSOFM. The final concentration of DMSO was 0.05%. As a control, only DMSO was added to the solution for activation medium and mSOFM at a concentration of 0.05%. Treatments were carried out with TSA at a concentration of 5 nM, 50 nM or 500 nM. The TSA-treated reconstructed embryos were washed with mSOFM twice 48 hours after the initiation of activation, transferred to 50-µl drops of the same culture medium at a density of 20 to 30 embryos per drop, and cultured at 39°C under 5% CO₂, 5% O₂ and 90% N₂ at saturation humidity.

(g) Assessment of embryo development
The cleavage rate was defined as the ratio of the number of cleaved embryos to the cultured embryos at 48 hours postactivation. The blastocyst development rate was defined as the ratio of the number of blastocysts to the cleaved embryos at 168 hours (7 days) postactivation. The cell number of blastocysts was determined by counting the number of nuclei after staining with Hoechst 33342 for 30 minutes.

(h) Statistics
The experiments were repeated three times. The cleavage rate, the blastocyst development rate and the cell number of blastocysts were analyzed with Fishers PLSD test following analysis of variance (ANOVA) (Stat View ver. 5.0, SAS Institute Ltd., Cary, NC, USA).

(3) Results
The effects of adding TSA to a culture medium on early development of bovine reconstructed embryos was examined. As a result, as shown in Table 6, TSA in the culture medium did not influence the cleavage rate (the ratio of the number of cleaved embryos to the cultured embryos (%)) or the cell number of blastocysts. However, the blastocyst development rate (the ratio of the number of embryos at the blastocyst stage to the cleaved embryos (%)) of 40% observed for the group of 50 nM TSA was higher than the rate of 19% observed for the group of no TSA addition (P<0.05). On the other hand, the blastocyst rate was lower with 500 nM TSA than with 5 or 50 nM TSA (7% vs 33% or 40%, respectively).

**Table 6: Effects of addition of TSA to a culture medium on early embryonic development of bovine reconstructed embry os^{a}**

| TSA conc. (nM) | Number of cultured embryos | Number of cleaved embryos (%)^{b} | Number of embryos at the blastocyst stage(%)^{c} | Cell number ± SEM |
|---|---|---|---|---|
| 0 | 103 | 70 (68) | 13 (19)^{d,e} | 87±9 |
| 5 | 106 | 85 (80) | 28 (33)^{d,f} | 89±6 |
| 50 | 97 | 73 (75) | 29 (40)^{f} | 83±6 |
| 500 | 97 | 73 (75) | 5 (7)^{e} | 92±8 |

| | | | | |
|---|---|---|---|---|
| a: Experiments were repeated three times. b: Cleavage rate (the ratio of the number of cleaved embryos to the cultured embryos (%)) is indicated in parentheses. c: The blastocyst development rate (the ratio of the number of embryos at the blastocyst stage to the cleaved embryos (%)) is indicated in parentheses. d-f: With significant difference between different marks (P<0.05). | | | | |

As described above, it was shown that the blastocyst development rate was increased by about 2-fold by exposing bovine cloned embryos to TSA at a certain concentration for a certain period of time.

### Industrial Applicability

The present invention provides a method by which a development rate in a somatic nuclear transfer technology or an artificial fertilization technology using a spermatid is increased.

## Claims

1. A method for producing a nuclear transfer oocyte, the method comprising:
(a) transferring a nucleus from a donor cell into an oocyte to obtain a nuclear transfer oocyte; and
(b) treating the nuclear transfer oocyte with an anti-methylation agent.

2. The method according to claim 1, wherein the treatment in step (b) is carried out during a period after the transfer of the nucleus and before embryo implantation.

3. The method according to claim 1, wherein the treatment in step (b) is carried out during a period after the transfer of the nucleus and before the end of the blastocyst stage.

4. The method according to claim 1, wherein the treatment in step (b) is carried out during a period after the transfer of the nucleus and before the end of the 32-cell stage.

5. The method according to claim 1, wherein the treatment in step (b) is carried out during a period after the transfer of the nucleus and before the end of the 4-cell stage.

6. The method according to claim 1, wherein the treatment in step (b) is carried out during a period after the transfer of the nucleus and before zygotic gene activation.

7. The method according to claim 1, wherein the treatment in step (b) is carried out during a period after the transfer of the nucleus and before the end of remethylation.

8. The method according to claim 1, wherein the anti-methylation agent is a histone deacetylase inhibitor.

9. The method according to claim 8, wherein the histone deacetylase inhibitor is trichostatin A or apicidin.

10. The method according to claim 1, wherein the oocyte is an enucleated oocyte.

11. The method according to claim 10, wherein the donor cell is selected from the group consisting of a cumulus cell, a fibroblast and an ES cell.

12. The method according to claim 1, wherein the donor cell is a spermatid.

13. A method for producing a cloned animal, the method comprising:
(a) transferring a nucleus from a donor cell into an oocyte to obtain a nuclear transfer oocyte;
(b) treating the nuclear transfer oocyte with an anti-methylation agent; and
(c) implanting the nuclear transfer oocyte treated with the anti-methylation agent into an animal.

14. A nuclear transfer oocyte produced according to the method defined by claim 1.
